# EUROPÄISCHE PATENTSCHRIFT

(11) **EP 1 087 768 B1**
(45) Veröffentlichungstag und Bekanntmachung des Hinweises auf die Patenterteilung: **14.01.2009**
(21) Anmeldenummer: 99926187.8
(22) Anmeldetag: 17.06.1999
(51) Int. Cl.: A61K 31/495

(54) **VERWENDUNG VON 1-(AMINOALKYL)-3-CHINOXALIN-2-ON-DERIVATEN ZUR HERSTELLUNG VON ALS ANTIOXIDANTIEN WIRKENDEN ZUSAMMENSETZUNGEN**
USE OF 1-(AMINOALKYL)-3-QUINOXALINE-2-ONE DERIVATIVES FOR THE PREPARATION OF COMPOUNDS HAVING AN ANTIOXIDANT ACTION
UTILISATION DE DERIVES DE 1-(AMINOALKYL)-3-QUINOXALIN-2-ONE POUR PRODUIRE DES COMPOSITIONS A ACTION ANTIOXYDANTE

(30) Priorität: 19.06.1998 AT 106398
(43) Veröffentlichungstag der Anmeldung: 04.04.2001
(73) Patentinhaber: PHAFAG AKTIENGESELLSCHAFT, 9496 Schaanwald (LI)
(72) Erfinder: EHRENBERGER, Klaus, A-1090 Wien (AT); FELIX, Dominik, CH-8008 Zürich (CH); KÖNIG, Peter, A-6800 Feldkirch (AT); POEGGELER, Burkhard, Catonsville, MD 21228 (US)
(74) Vertreter: Cunow, Gerda
(86) Internationale Anmeldenummer: PCT/AT1999/000158
(87) Internationale Veröffentlichungsnummer: WO 1999/066931

(56) Entgegenhaltungen:
- EP-A- 0 542 689
- EHRENBERGER K ET AL: "Caroverine depresses the activity of cochlear glutamate receptors in guinea pigs: in vivo model for drug-induced neuroprotection?." NEUROPHARMACOLOGY, (1992 DEC) 31 (12) 1259-63. , XP002116179
- SALETU B ET AL: "On the cerebro-protective effects of caroverine, a calcium-channel blocker and antiglutamatergic drug: double-blind, placebo-controlled, EEG mapping and psychometric studies under hypoxia." BRITISH JOURNAL OF CLINICAL PHARMACOLOGY, (1996 FEB) 41 (2) 89-99. , XP002116180
- SALETU B (REPRINT) ET AL: "ACUTE CENTRAL EFFECTS OF THE CALCIUM-CHANNEL BLOCKER AND ANTIGLUTAMATERGIC DRUG CAROVERINE - DOUBLE-BLIND, PLACEBO-CONTROLLED, EEG MAPPING AND PSYCHOMETRIC STUDIES AFTER INTRAVENOUS AND ORAL-ADMINISTRATION" ARZNEIMITTEL-FORSCHUNG/DRUG RESEARCH, (MAR 1995) VOL. 45-1, NO. 3, PP. 217-229. ISSN: 0004-4172., XP002116181 UNIV VIENNA, SCH MED, DEPT PSYCHIAT, DIV PHARMACOPSYCHIAT & SLEEP RES, WAHRINGER GURTEL 18-20, A-1090 VIENNA, AUSTRIA (Reprint);LANDES NERVENKRANKENHAUS VALDUNA, DEPT PSYCHIAT 1, RANKWEIL, AUSTRIA
- MENKE H. ET AL: "[Calcium antagonist in the cytostatic treatment of malignant tumours]. CALCIUM-ANTAGONISTEN IN DER ZYTOSTATIKATHERAPIE MALIGNER TUMOREN." DEUTSCHE MEDIZINISCHE WOCHENSCHRIFT, (1988) 113/44 (1728-1732). , XP002116182
- TSURUO T. ET AL: "Potentiation of vincristine and adriamycin effects in human hemopoietic tumor cell lines by calcium antagonists and calmodulin inhibitors." CANCER RESEARCH, (1983) 43/5 (2267-2272). CODEN: CNREA8, XP002116183
- NASPITZ C.K.: "Use of calcium channel blocking agents in the management of childhood asthma." JOURNAL OF ASTHMA, (1984) 21/6 (451-460). CODEN: JOUADU, XP002116184
- KAIK G. ET AL: "[Inhalation or peroral therapy with bronchospasmolytics]. INHALATIVE ODER PERORALE THERAPIE MIT BRONCHOSPASMOLYTIKA." WIENER MEDIZINISCHE WOCHENSCHRIFT, (1977) 127/1 (22-28). CODEN: WMWOA4, XP002116185
- DATABASE CHEMABS [Online] CHEMICAL ABSTRACTS SERVICE, COLUMBUS, OHIO, US MITSUBISHI CHEMICAL INDUSTRIES CO., LTD., JAPAN: "Enhancement of neoplasm inhibitor activity by quinoxaline derivatives" retrieved from STN Database accession no. 99:16559 XP002116981 & JP 58 057317 A (MITSUBISHI CHEMICAL INDUSTRIES CO., LTD., JAPAN) 5. April 1983 (1983-04-05)

## Beschreibung

Die vorliegende Erfindung bezieht sich auf die Verwendung von 1-(Aminoalkyl)-3-chinoxalin-2-on-Derivaten, insbesondere 1-Diethylaminoethyl-3-chinoxalin-2-on-Derivaten der Formel 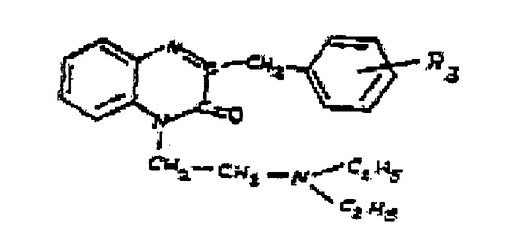 worin R₃ Hydroxy, Methoxy, Ethoxy oder Wasserstoff bedeutet, oder pharmazeutisch verträglichen Salzen davon, für die Herstellung von neuartigen pharmazeutischen Zusammensetzungen.

Die pharmazeutische Wirkung von 1-(Aminoalkyl)-3-chinöxalin-2-on-Derivaten ist seit vielen Jahren bekannt und insbesondere 1-Diethylaminoethyl-3-(p-methoxybenzyl)-1,2-dihydrochinoxalin-2-on, dessen Nicht-Nomenklaturname Caroverin ist, wurde bereits als wirksames Spasmolytikum im Gastro-Intestinal-Bereich eingesetzt. Die Wirksamkeit dieser Substanz wird hiebei insbesondere ihren Calciumblockierenden Eigenschaften zugeschrieben, wobei sie die Calcium-mediatisierte Aktivierung der myofibrillaren ATP blockiert.

Die EP-A 0 032 564 beschreibt die Verwendung von Caroverin oder dessen pharmazeutisch unbedenklichen Salzen für eine Vielzahl von Herz-Kreislauferkrankungen und als Präparat zur Hemmung der Plättchenaggregation im menschlichen Blut, als durchblutungssteigerndes Präparat, aber auch als Substanz zur Behandlung von angina pectoris, Myocardinfarkten, hypertonen Zuständen und dgl. Die Wirkung des Caroverins wurde in dieser EP-A 0 032 564 darauf zurückgeführt, daß es ein spezifischer Ca-Antagonist ist und daß Caroverin fähig ist, bis zu 50 % des Flusses der Ca₂⁺-Ionen in die Zellen zu inhibieren.

Möslinger beschrieb bereits in Subsidia med. 22, 3, Seiten 78 - 85 (1970), daß Caroverin epileptische Anfälle unterdrücken kann. Auch die Verwendung von Caroverin bei der Behandlung von akuten Alkohol-Entzugserscheinungen und sein Einsatz bei Alkaloid-Entzugserscheinungen wurden bereits beschrieben.

Der EP-A 0 542 689 ist die Verwendung von 1-(Aminoalkyl)-3-chinoxalin-2-on-Derivaten als neuroprotektive Substanzen zu entnehmen, wobei insbesondere deren Verwendung in Glutamat-induzierten und Glutamat-Rezeptor-mediatisierten, neurotoxischen Dysfunktionen und funktionellen Störungen des zentralen Nervensystems und insbesondere des Innenohrs und der Retina beschrieben ist. In dieser Literaturstelle ist auch der Einsatz dieser Substanzen zur Behandlung von degenerativen Prozessen von Neuronen im Zentralnervensystem, nämlich Alzheimer, Huntington's Erkrankung, Parkinson-Erkrankung, Wernicke-Korsakoff und Jakob-Creutzfeld-Syndrom, angesprochen. Die Wirkung der speziellen, genannten Chinoxalin-Derivate wird der selektiven Blockierung der NMDA- und der nicht-NMDA-Rezeptoren ohne Beeinflussung von anderen Rezeptoren zugeschrieben, so daß die in dieser Literaturstelle beschriebenen Pharmazeutika nur zur Anwendung in Glutamat-induzierten und Glutamat-Rezeptor-mediatisierten, neurotoxischen Dysfunktionen, nämlich Funktionsstörungen des Innenohrs und der Retina, und für die angesprochenen, degenerativen Prozesse einsetzbar erscheinen.

Dem Derwent Abstract, Publikationsnr. AN 83-45789 K ist eine Wirkung von Caroverin, insbesondere von 1-(2-Dialkylaminoalkyl)-3-(p-alkoxybenzyl)-1,2-dihydrochinoxalin-2-on-Substanzen als Wirksubstanz bei der Steuerung des Inflow und Outflow von Antikrebsmitteln zu entnehmen, wobei hier die Antikrebswirkung entwickelt wird, wenn die Antikrebsmittel durch die Zellmembranen in die Zellen transferiert werden. Bei üblichen Antikrebsmitteln wird die Wirkung im Laufe der Zeit schwächer, da die Krebszellen einerseits gegen sie resistent werden, andererseits die Antikrebsmittel aus den Zellen ausströmen bzw. langsam austreten, was gemäß diesem Stand der Technik durch die Gabe von Chinoxalinderivaten verhindert wird, da diese Chinoxalinderivate das Antikrebsmittel am Ausfließen aus den Zellen und die Beibehaltung der hohen Konzentration des Antikrebsmittels in den Zellen gewährleisten.

Die DE-A 25 21 905 beschreibt ein Arzneimittel, welches 1-Diethylaminoethyl-3-(p-methoxybenzyl)-2,1,2-dihydrochinoxalin-2-on enthält, welches Effekte auf die zerebralgefäße bzw. die zerebrale Durchblutung zeigen soll.

Aus JAOCS J Am Oil Chem Soc, Feb 1998, Vol. 75, 2, 199-218, ist eine Auflistung bekannt geworden, welcher Krankheiten entnehmbar sind, in welchen freie Radikale des Sauerstoff-Zellmetabolismus involviert sind. Schließlich ist der US-A 4,339,452 und der CH-B 653 254 entnehmbar, daß mit Caroverin hischämische Herzkrankheiten ebenso wie tachyocarde Herzrhythmusstörungen und hypotone Zustände behandelt werden können.

Die vorliegende Erfindung zielt nun darauf ab, neben der eingangs genannten Verwendung von spezifischen 1-(Aminoalkyl)-3-chinoxalin-2-on-Derivaten neuartige pharmazeutische Einsatzgebiete zur Verfügung zu stellen, und betrifft daher die Verwendung von 1-(Aminoalkyl)-3-chinoxalin-2-on-Derivaten, insbesondere von 1-Diethylaminoethyl-3-chinoxalin-2-on-Derivaten der Formel 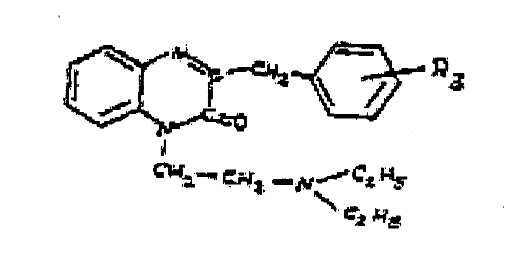 worin R₃ Hydroxy, Methoxy, Ethoxy oder Wasserstoff bedeutet, oder pharmazeutisch verträglichen Salzen davon, für die Herstellung von antioxidativen Zusammensetzungen zur Behandlung von durch freie Radikale des Sauerstoff-Zellmetabolismus hervorgerufenen Erkrankungen, gewählt aus der Gruppe von durch freie OH-Radikale hervorgerufenen Veränderungen der DNA, nämlich Krebs, von durch freie Radikale des Sauerstoff-Zellmetabolismus hervorgerufenen Zellalterungserscheinungen und/oder Zellzerstörungen, nämlich Hautalterungserscheinungen, von durch freie OH-Radikale und/oder freie ONOO-Radikale hervorgerufenen Typ 1-Allergien, und zur Stimulation des Wachstums von Nervenzellen, wie glutamatergen Nervenzellen, gewählt aus der Gruppe Wiederherstellung der Funktionsfähigkeit von Nerven nach einem Hirnschlag, Verbesserung von Schluckstörungen, Artikulationsstörungen, nämlich Sprachstörungen und Querschnittslähmungen, Behandlung von Schizophrenie oder der amyotrophen Lateralsklerose, Stimulation des glutamatergen Nervenwachstums bei einem Kochleaimplantat, Funktionsausfälle des Hörnervs, Gleichgewichtsnervs, Riechnervs oder Gesichtsnervs,
wobei das 1-(Aminoalkyl)-3-chinoxalin-2-on-Derivat oder seine pharmazeutisch verträglichen Salze in einer täglichen Dosis von 5 bis 15 mg/kg Körpergewicht eingesetzt wird. Bei ausgedehnten Studien betreffend den Aufbau der Moleküle und ihrer möglichen Wirkungsweise wurde von der Anmelderin gefunden, daß die erfindungsgemäß verwendeten 1-Diethylaminoethyl-3-chinoxalin-2-on-Derivate extrem starke Antioxidantien sind und daß sie daher insbesondere eine extrem hohe Kapazität als Radikalenfänger vor allem für die für die Verursachung einer Vielzahl von Erkrankungen verantwortlichen Hydroxyl-, Peroxyl- und Peroxynitrit-Radikalen aufweisen. Die Wirkung als Antioxidantien und somit als Radikalenfänger wird auf die strukturellen Gegebenheiten in diesen speziellen Substanzen zurückgeführt, welche Chinoxaline mit elektronenreichen, aromatischen Ringsystemen mit redoxaktiven Sauerstoff- und Stickstoffatomen sind. Aufgrund des hohen Elektronenangebots in dem Molekül konnte nachgewiesen werden, daß die Verbindungen in der Lage sind, freie Radikale zu binden, so daß durch freie Radikale hervorgerufene Erkrankungen und insbesondere durch freie Radikale des Sauerstoff-Zellmetabolismus hervorgerufene Erkrankungen durch die speziellen, erfindungsgemäß verwendeten 1-Diethylaminoethyl-3-chinoxalin-2-on-Derivate behandelt werden können bzw. deren Ausbruch verhindert werden kann. Darüber hinaus hat sich überraschenderweise gezeigt, daß spezielle 1-(Aminoalkyl)-3-chinoxalin-2-on-Derivate, insbesondere Caroverin, fähig sind, metabotrope Glutamatrezeptoren zu antagonisieren. Da die Glutamatrezeptoren, insbesondere die metabotropen Glutamatrezeptoren, als die Förderer bzw. Antreiber des intrazellulären Stoffwechsels erkannt wurden, kann insbesondere dieser intrazelluläre Stoffwechsel durch Caroverin und andere 1-(Aminoalkyl)-3-chinoxalin-2-on-Derivate antagonisiert, d.h. deutlich verzögert, werden.

Aufgrund ihrer starken antioxidativen Wirkung und somit der Radikalenfängereigenschaften sind die erfindungsgemäß verwendeten 1-(Aminoalkyl)-3-chinoxalin-2-on-Derivate insbesondere zur Behandlung von durch freie OH-Radikale hervorgerufenen Veränderungen der DNA, nämlich Krebs, wirksam. Diese Wirkungsweise ist darauf zurückzuführen, daß eine der hauptsächlichen, biologischen Wirkungen von insbesondere OH-Radikalen und ONNO-Radikalen die Oxidation von DNA, mit der biologischen Konsequenz einer Mutation derselben und in der Folge der Ausbildung von Krebs ist. Diese Wirkungsweise von insbesondere OH-Radikalen ist in einer Vielzahl von Studien untersucht und nachgewiesen worden. Aus diesen Studien ergibt sich, daß aufgrund der onkogenen Wirkung des OH-Radikals auf die DNA ein potenter OH-Radikalenfänger, d.h. ein Antioxidationsmittel, welches fähig ist, die oxidative Wirkung des OH-Radikals auf biologisches Material zu hemmen, als Mittel zur Behandlung von Krebserkrankungen geeignet ist, wie dies erfindungsgemäß vorgeschlagen wird.

Aufgrund der starken antioxidativen Eigenschaften der erfindungsgemäß verwendeten 1-(Aminoalkyl)-3-chinoxalin-2-on-Derivate hat sich deren Einsatz bei von durch freie Radikale des Sauerstoff-Zellmetabolismus hervorgerufenen Zellalterungserscheinungen und/oder Zellzerstörungen, nämlich Hautalterungserscheinungen, als äußerst effizient und erfolgreich herausgestellt. Diese Wirkung tritt sowohl bei einem durch Chemikalien bewirkten, oxidativen Angriff auf Körperzellen als auch bei Hautzellen, welche beispielsweise durch Umwelteinflüsse, insbesondere Sonnenbestrahlung, einem starken, oxidierenden Angriff ausgesetzt sind, ein.

Allergische Reaktionen, insbesondere Typ 1-Allergien werden beispielsweise durch den Angriff von Histamin, Cytokinen, Lipidmediatoren und Neuromediatoren hervorgerufen und gegenwärtig durch die Verabreichung von Antihistaminika behandelt. Es wurde nun überraschenderweise gefunden, daß bei Einwirkung von Histaminen, Cytokinen, Lipidmediatoren und Neuromediatoren Peroxynitrit- und OH-Radikale freigesetzt werden und daß durch Einsatz der erfindungsgemäß verwendeten 1-(Aminoalkyl)-3-chinoxalin-2-on-Derivate aufgrund ihrer antioxidativen Eigenschaften und insbesondere Radikalenfängereigenschaften das Auftreten von Sekundärwirkungen der Allergien, wie z.B. Entzündungen und dgl., vollständig vermieden werden können und auch die Primärerscheinungen, wie z. B. Niesen, nahezu unmittelbar nach der Verabreichung der erfindungsgemäß verwendeten 1-(Aminoalkyl)-3-chinoxalin-2-on-Derivate gestoppt werden können.

Im Zuge der Studien wurde, da von den erfindungsgemäß verwendeten Chinoxalin-2-on-Derivaten bekannt ist, daß sie in der pharmazeutischen Anwendung ohne merkbare Nebenwirkungen angewandt werden können, versucht, durch Erhöhung der Verabreichungsdosis den Wirkungsbereich der Substanz als Antioxidationsmittel bzw. als Radikalenfänger abzugrenzen bzw. zu optimieren. Überraschenderweise wurde im Zuge dieser Studien festgestellt, daß die erfindungsgemäß verwendeten 1-(Aminoalkyl)-3-chinoxalin-2-on-Derivate neben ihrer bekannten Wirkung als neuroprotektive Substanzen und aufgrund der im Zuge der zur vorliegenden Erfindung führenden Studien herausgefundenen, antioxidativen Wirkung eine neuroregenerative Wirkung zeigen, wenn sie in höheren Dosen verabreicht werden. Dies deshalb, da Radikale die intrinsischen Neurotrophine blockieren, so daß, wenn nun ein Radikalenfänger eingesetzt wird, die Neurotrophinaktivität wiederhergestellt wird und die neuroregenerative Wirkung der erfindungsgemäß verwendeten Substanzen einsetzt. Es war daher im Zuge der Studien möglich, geschädigte Nerven und insbesondere im Zuge von degenerativen Prozessen von Neuronen im zentralen Nervensystem angegriffene bzw. zerstörte Nervenbahnen nicht nur vor weiterer Zerstörung zu schützen, wie dies bereits in der EP-B 0 542 689 behauptet ist, sondern die zerstörten Nervenbahnen soweit rückzubilden, so daß neben der bekannten neuroprotektiven Wirkung derartiger Substanzen überraschenderweise auch eine neuroregenerative Wirkung eintrat, welche zu einer merkbaren Verringerung der Symptome der neurodegenerativen Erkrankungen und zu einer Besserung des Zustandes der Patienten führt.

Eine besonders überraschende und therapeutisch besonders interessante Einsatzmöglichkeit der erfindungsgemäß verwendeten 1-(Aminoalkyl)-3-chinoxalin-2-on-Derivate ist dadurch gegeben, daß sie zur Stimulation des Wachstums der Nervenzellen, gewählt aus der Gruppe Wiederherstellung der Funktionsfähigkeit von Nerven nach einem Hirnschlag, Verbesserung von Schluckstörungen, Artikulationsstörungen, nämlich Sprachstörungen und Querschnittslähmungen, Behandlung von Schizophrenie oder der amyotrophen Lateralsklerose, zur Stimulation des glutamatergen Nervenwachstums bei einem Kochleaimplantat, Funktionsausfällen des Hörnervs, Gleichgewichtsnervs, Riechnervs oder Gesichtsnervs geeignet sind.

Im Zuge von Untersuchungen, in welchen Dosen die erfindungsgemäß verwendeten Chinoxalin-Derivate pharmazeutisch ohne gravierende Nebenwirkungen an Patienten verabreicht werden können, wurde, wie oben bereits angedeutet, überraschenderweise festgestellt, daß die Substanzen neben den bekannten neuroprotektiven Wirkungen insbesondere neuroregenerative Wirkungen zeigen, so daß eine Verwendung der erfindungsgemäßen Zusammensetzungen bei auf der Degeneration von insbesondere glutamatergen Nervenzellen beruhenden Erkrankungen nicht nur ein Stationärhalten des Krankheitsbilds, wie dies im Stand der Technik gezeigt ist, ermöglicht, sondern erfindungsgemäß in jedem Fall eine deutliche Verbesserung desselben sowie ein neuerliches Wachstum von Nervenzellen erzielt werden kann.

So wurde insbesondere bei Patienten, welche ein Kochleaimplantat tragen, welches zur Stimulation der Nerven im Innenohr dient, festgestellt, daß bei gleichzeitiger Verabreichung von den erfindungsgemäß verwendeten Chinoxalin-2-on-Derivaten und insbesondere von Caroverin der durch das Implantat stimulierte Nerv zu einem Teil regeneriert wurde und dadurch die Kontaktierung des Implantates deutlich verbessert wurde, was die Hörleistung der so behandelten Patienten deutlich steigert.

Von den erfindungsgemäß verwendeten 1-(Aminoalkyl)-3-chinoxalin-2-on-Derivaten zeigten insbesondere das Hydroxy-Derivat 1-Diethylaminoethyl-3-(p-hydroxybenzyl)-1,2-dihydrochinoxalin-2-on oder das Methoxy-Derivat 1-Diethylaminoethyl-3-(p-methoxybenzyl)-1,2-dihydrochinoxylin-2-on (Caroverin) eine gute Wirkung und konnten bei in-vitro-Studien auch in höheren Dosierungen ohne schädliche Nebenwirkungen an den untersuchten Zellen bzw. bei in-vivo-Studien an Versuchstieren und Patienten ohne schädliche Nebenwirkungen verabreicht werden.

Es hat sich insbesondere als vorteilhaft erwiesen, daß die 1-(Aminoalkyl)-3-chinoxalin-2-on-Derivate oder ihre pharmazeutisch verträglichen Salze bei ihrem Einsatz als Antioxidantien und insbesondere als Radikalenfänger in einer täglichen Dosis von 5 bis 10 mg/kg Körpergewicht eingesetzt werden. Bei Verabreichung einer derartigen Dosis gelingt es, die Krankheitssymptome deutlich zu verringern bzw. zu mildern, ohne daß gleichzeitig Nebenwirkungen aufgrund der Dosierung des Medikaments eintreten.

Bei niedrigen Dosierungen und insbesondere bei aus der Literatur bekannten Dosierungen der Substanzen und insbesondere von Caroverin als neuroregenerative Substanz, als Calcium-Antagonist oder auch als antioxidative Substanz, wie dies erfindungsgemäß vorgeschlagen wird, zeigte sich bei Versuchen, insbesondere in-vitro-Versuchen, keinerlei neuroregenerative Wirkung der Substanz, sodaß für das Erreichen der erfindungsgemäß erzielbaren, neuroregenerativen Wirkung eine hohe Dosis der Substanz angewandt werden muß. Bei entsprechenden Versuchen wurde festgestellt, daß auch die erfindungsgemäß vorgeschlagenen, hohen Dosierungen nahezu nebenwirkungsfrei an Patienten verabreicht werden können.

Die erfindungsgemäß verwendeten 1-(Aminoalkyl)-3-chinoxalin-2-on-Derivate können in jeder bekannten Formulierung verabreicht werden und insbesondere oral, transdermal, topisch und parenteral, wobei mit Ausnahme der Anwendung als Mittel zur Verhinderung der vorzeitigen Alterung von Hautzellen die intravenöse Verabreichung bevorzugt erscheint.

Die Erfindung wird nachfolgend anhand von Versuchen bzw. Ausführungsbeispielen unter Bezugnahme auf die beiliegenden Zeichnungen näher erläutert, in welchen:
Fig. 1 ein Tonaudiogramm eines weiblichen Patienten zeigt;
Fig. 2 ein Tonaudiogramm eines männlichen Patienten zeigt;
Fig. 3 ein Tonaudiogramm eines weiteren männlichen Patienten zeigt;
Fig. 4 ein Sprech- bzw. Artikulationsdiagramm eines männlichen Patienten zeigt;
Fig. 5 die Inhibition des ABTS-Kationenradikals durch Caroverin zeigt; und
Fig. 6 vergleichskinetische Studien betreffend die Reaktivität von Caroverin gegenüber Radikalen zeigt.

### Beispiel 1, 2 und 3

Anhand von klinischen Beispielen wurde die Wirksamkeit von Caroverin (1-Diethylaminoethyl-3-(p-methoxybenzyl)-1,2-dihydrochinoxalin-2-on) als neuroregenerative Substanz bei zwei Patienten, die einen massiven Hörsturz erlitten, getestet.

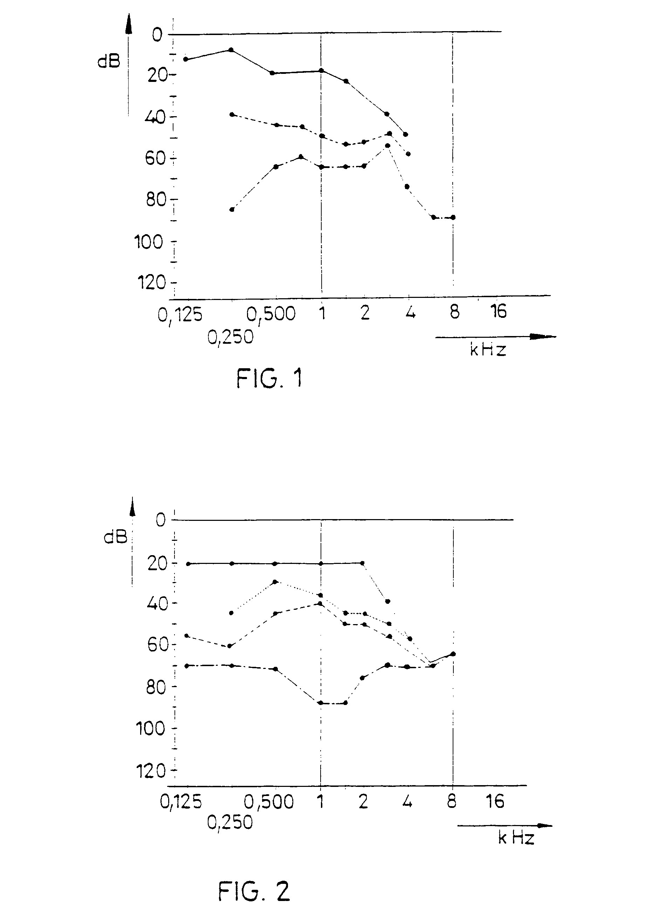

Im beiliegenden Diagramm von Fig. 1 ist das Tonaudiogramm eines weiblichen Patienten gezeigt, wobei die strichpunktierte Linie das Tonaudiogramm nach einem Hörsturz darstellt, die strichlierte Linie das Tonaudiogramm unmittelbar nach dem Einsatz der Behandlung zeigt und die durchgezogene Linie das Tonaudiogramm der Patientin zwei Wochen nach dem Beginn der Behandlung, wobei die Patientin in diesem Zeitraum durch Verabreichung von erhöhten Dosen Caroverin behandelt wurde. Es wurde hiebei dreimal täglich je 240 mg Caroverin, gelöst in 250 ml physiologischer Kochsalzlösung, langsam intravenös verabreicht.

Die deutliche Erhöhung der Hörleistung der Patientin ist auf die wenigstens teilweise Rückbildung des Hörnervs zurückzuführen.

Fig. 2 zeigt ein analoges Beispiel anhand eines männlichen Patienten, wobei wiederum die strichpunktierte Linie das Tonaudiogramm des Patienten nach dem Hörsturz und vor Beginn einer Behandlung und vor einem Einsetzen eines Kochleaimplantats zeigt, die strichlierte Linie das Hördiagramm des Patienten nach Verabreichung niedriger Dosen Caroverin zeigt, die punktierte Linie das Hördiagramm des Patienten nach zweiwöchiger Behandlung durch Verabreichung von erhöhten Dosen an Caroverin zeigt und die durchgezogene Linie wiederum das Tonaudiogramm des Patienten nach zweiwöchiger Verabreichung von hohen Dosen Caroverin und nach Einsetzen eines Kochleaimplantats zeigt. Aus Fig. 2 ist deutlich ersichtlich, daß die Verabreichung von niedrigen Dosen Caroverin, d.h. dreimal 160 mg Caroverin täglich, die Hörleistung des Patienten zu verbessern vermag, wobei diese Verbesserung jedoch wenig signifikant ist. Wenn dem Patienten viermal täglich 240 mg/250 ml physiologischer Kochsalzlösung Caroverin über einen Zeitraum von zwei Wochen verabreicht werden und zusätzlich ein Kochleaimplantat eingesetzt wird, wird eine stabile Hörleistung im Frequenzbereich zwischen 0,125 und 2 kHz erreicht, was nahezu der Hörleistung eines Gesunden entspricht.

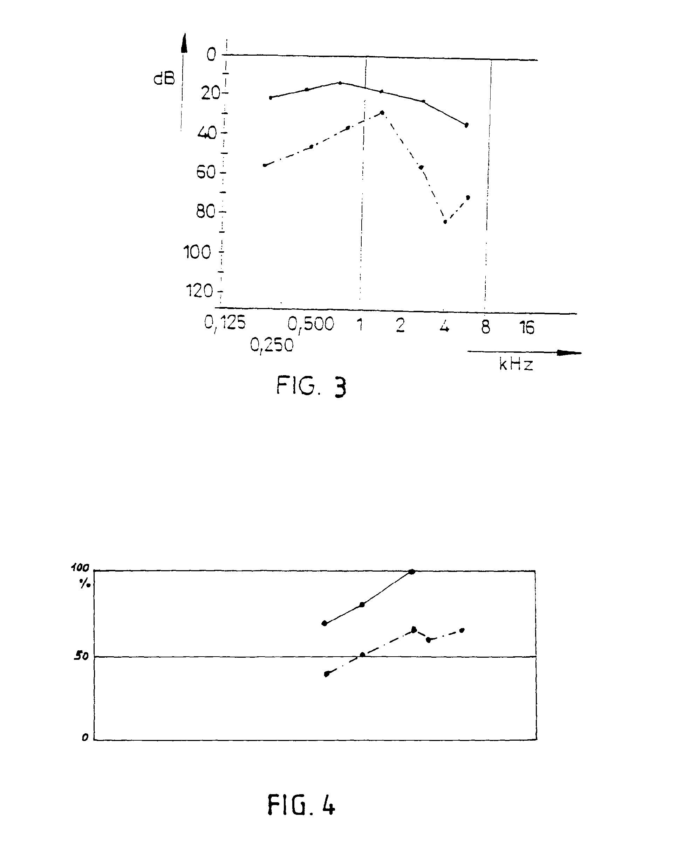

Fig. 3 zeigt ein weiteres Beispiel anhand eines männlichen Patienten, wobei wiederum die strichpunktierte Linie das Tonaudiogramm des Patienten nach dem Hörsturz und vor dem Beginn einer Behandlung zeigt und die durchgezogene Linie das Diagramm des Patienten nach zweiwöchiger Behandlung mit erhöhten Dosen von Caroverin zeigt. Ein Kochleaimplantat wurde nicht eingesetzt. Aus Fig. 3 ist deutlich ersichtlich, daß durch die Verabreichung von erhöhten Dosen Caroverin, wobei im vorliegenden Fall täglich 300 mg in 500 ml physiologischer Kochsalzlösung Caroverin über einen Zeitraum von 2 Wochen verabreicht werden, eine stabile Hörleistung im Frequenzbereich zwischen 0,250 und 8 kH erreicht werden kann, was im wesentlichen der Hörleistung eines Gesunden entspricht.

Aus den Daten der Fig. 1, 2 und 3 ist deutlich ersichtlich, daß Caroverin in erhöhten Dosen gegenüber den bekannten Verabreichungen neuroregenerativ zu wirken vermag und daß insbesondere Patienten, welche zusätzlich ein Kochleaimplantat tragen, aufgrund der besseren Kontaktierung des Implantats mit dem Hörnerv deutlich verbesserte Hörleistungen zeigen.

### Beispiel 4 (welches nicht von der Anmeldung umfaßt ist)

Anhand von zwei männlichen Patienten im Alter zwischen 70 und 75 Jahren, welche an der Alzheimer'schen Erkrankung leiden, wurde die Wirkung von Caroverin als neuroregenerative Substanz weiter untersucht. Die Patienten hatten ein durchschnittliches Körpergewicht von 75 kg und sie wurden stationär behandelt. Den Patienten wurde jeweils dreimal täglich 400 mg Caroverin in physiologischer Kochsalzlösung als Infusion über einen Zeitraum von etwa 2 h verabreicht, wobei diesen Infusionslösungen übliche Zusatzbestandteile beigemischt sein konnten. Die Behandlungsdauer wurde in jedem Fall für drei Wochen beibehalten.

Vor Beginn der Behandlung mit erhöhten Dosen an Caroverin waren die Patienten vergeßlich, erkannten ihre nächsten Verwandten nicht und galten als Pflegefälle. Am Ende der Behandlungsdauer von drei Wochen war bei den zwei Patienten eine deutliche Verbesserung des Zustandes zu bemerken, sie konnten teilweise wiederum selbständig essen und erkannten ihre nächsten Verwandten bzw. das Pflegepersonal. Mit Rücksicht auf die Tatsache, daß bekannte Substanzen, die zur Behandlung der Alzheimer'schen Erkrankung verwendet werden, lediglich eine Stabilisierung des Zustands mit sich bringen können, ist die Wirkung von Caroverin offensichtlich auf die neuroregenerative Wirkung der Substanz zurückzuführen, sodaß innerhalb einer relativ kurzen Verabreichungszeit gewisse neurologisch gesteuerte Funktionen in einem gewissen Ausmaß wiedererlernt werden konnten.

Auch bei intravenöser Verabreichung von dreimal täglich je 160 mg Caroverin in 250 ml Kochsalzlösung über einen Zeitraum von drei Wochen konnten Verbesserungen des Zustandes der Patienten erzielt werden, wobei diese jedoch nicht so deutlich und langandauernd beobachtbar waren, woraus unmittelbar geschlossen werden kann, daß nachhaltige therapeutische Erfolge nur erzielt werden können, wenn die neuroregenerative Wirkung von Caroverin eintritt.

### Beispiel 5

4 Patienten, zwei weibliche und zwei männliche, im Alter zwischen 40 und 50 Jahren, die an periodischer Schizophrenie leiden, wurden während massiver Krankheitsschübe mit erhöhten Dosen an Caroverin behandelt. Es wurden ihnen jeweils dreimal täglich je 400 mg Caroverin in physiologischer Kochsalzlösung als Infusion über einen Zeitraum von etwa 30 min verabreicht. Die Behandlungsdauer betrug 7 Tage, 14 Tage bzw. 21 Tage.

Unmittelbar nach Beginn der Behandlung mit erhöhten Dosen an Caroverin zeigen sämtliche vier Patienten eine deutliche Verbesserung in ihrem Verhalten. Sie zeigten eine merkbare Aufhellung, kaum Halluzinationen und waren in der Lage, sowohl reale Gegebenheiten zu erkennen und ihnen entsprechend zu handeln, waren kooperativ als auch Verrichtungen des täglichen Bedarfs ohne größere Anstrengungen und Orientierungsstörungen durchzuführen.

Bei allen vier Patienten zeigte sich, daß über die Dauer der Behandlung diese Verbesserungen des Zustands anhielten, jedoch konnten nur bei dem Patienten, bei welchem die Behandlungsdauer 21 Tage betrug, auch über die Behandlungsdauer hinaus Erfolge erzielt werden.

Im Vergleich zu bestehenden Behandlungsmethoden zeigte sich während der Dauer der Behandlung eine signifikante Verbesserung im Verhalten der Patienten, wobei darüber hinaus die massiven Nebenwirkungen, welche üblicherweise bei der Verabreichung von Psychopharmaka eintraten, wie Antriebslosigkeit, Gewichtszunahme und dgl., bei der Verabreichung von Caroverin hintangehalten werden konnten.

Durch Verabreichung von täglich 240 mg Caroverin bzw. 400 mg Caroverin in Kombination mit 10 mg Haloperidol (einem Neuroleptikum aus der Butyrophenonklasse) konnten analoge Ergebnisse wie bei der höher dosierten Verabreichung von Caroverin alleine erzielt werden, wobei die Verbesserungen des Zustandes nach dem Absetzen von Caroverin nicht anhielten.

### Beispiel 6

Die neuroregenerative Wirkung von Caroverin wurde weiters anhand von zwei Patienten, welche an einer Lähmung des Gesichtsnervs litten, untersucht.

Zwei weibliche Patienten, welche an einer massiven Lähmung des Trigeminusnervs litten, wurden über einen Zeitraum von 4 Wochen mit Caroverin in Form einer kombinierten Kur behandelt, wobei einerseits Caroverin durch intravenöse Verabreichung von jeweils 360 mg Caroverin, dreimal täglich, verabreicht wurde und darüberhinaus der Wirkstoff in Form eines Depotpflasters, enthaltend 1000 mg Caroverin, verabreicht wurde. Das Depotpflaster wurde einmal wöchentlich getauscht. Bereits nach 2 Wochen konnte bei jeder der Patientinnen eine Verbesserung der Lähmungserscheinungen dahingehend beobachtet werden, daß die gelähmte Gesichtshälfte wiederum Ansätze einer Mimik zeigte und daß die Patientinnen insbesondere die Augenlider wiederum nach Belieben beherrschen konnten.

Eine weitere Verlängerung der Behandlungsdauer mit Caroverin ergab weitere Verringerungen der Lähmungserscheinungen, wobei sich jedoch herausstellte, daß eine vollkommene Wiederherstellung der Funktion des Gesichtsnervs lediglich durch Behandlung mit Caroverin nicht ohne weiteres erzielt werden konnte.

### Beispiel 7

Bei einem an massiven Sprachstörungen infolge eines Schlaganfalles leidenden Patienten wurde Caroverin über einen Zeitraum von 3 Monaten in einer erhöhten Dosis von 4 x tägl. 160 mg Caroverin in physiologischer Kochsalzlösung intravenös verabreicht. Wie aus der beiliegenden Fig. 4 ersehen werden kann, hatte der Patient zu Beginn der Behandlung, was im Diagramm mit strichpunktierter Linie zu erkennen ist, eine in etwa 50 %-ige Artikulationsfähigkeit, wobei er nach Ende der Behandlung seine 100 %-ige Artikulationsfähigkeit wiedererlangt hat (durchgezogene Linie in Fig. 4) und Sprachstörungen nicht mehr beobachtet werden konnten. In bezug auf die Rückbildung der Sprachstörungen wurde nachgewiesen, daß sich die entsprechenden Nerven, welche für die Artikulationsfähigkeit verantwortlich sind, aufgrund der neuroregenerativen Wirkung von Caroverin rückgebildet haben und somit eine vollständige Wiedererlangung der Sprachfähigkeit erzielt werden konnte.

### Beispiel 8

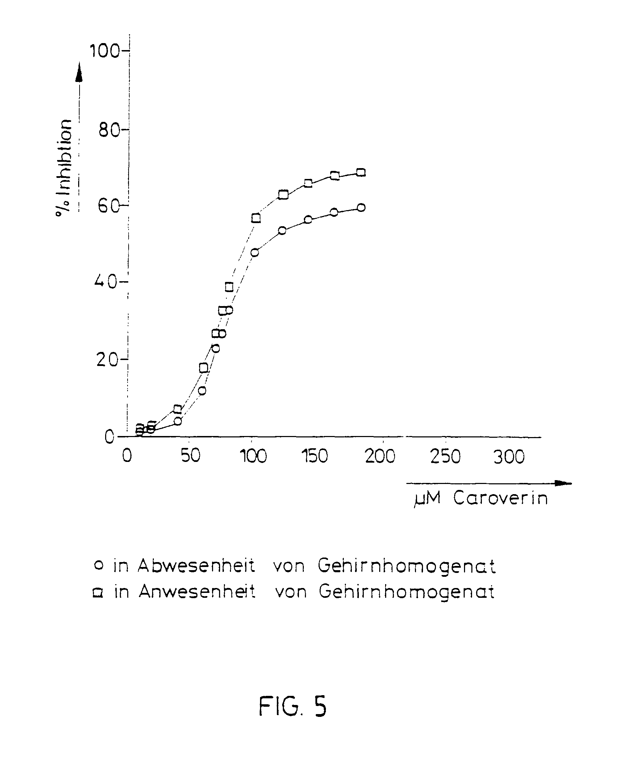

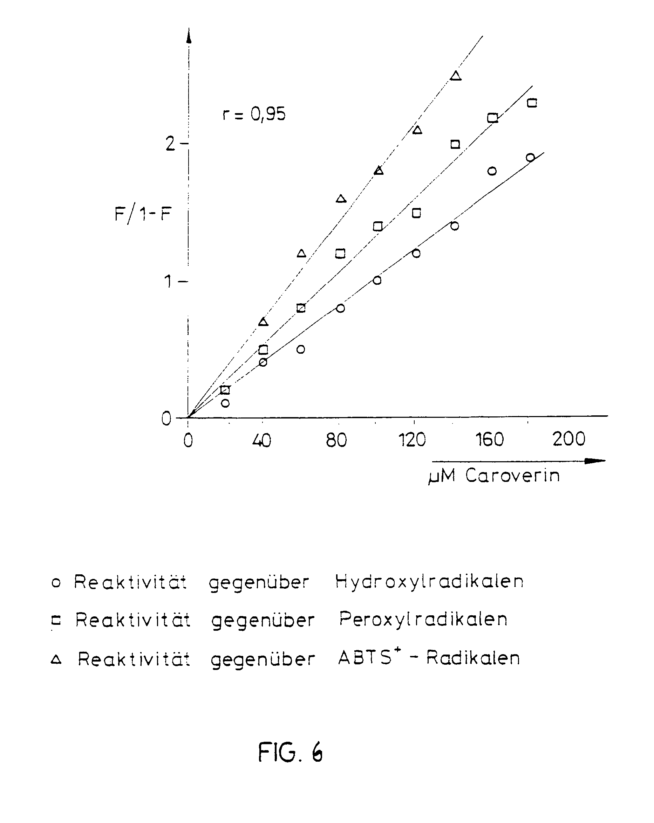

In bezug auf die antioxidative Wirkung von 1-(Aminoalkyl)-3-chinoxalin-2-on-Derivaten und in bezug auf deren Radikalfängereigenschaften wurde in-vitro-Versuche mit Caroverin und dem spezifischen, radikalfangenden Reagenz 2,2'-Azino-bis(3-ethylbenz)-thiazolin-6-sulfonsäure) (ABTS) durchgeführt, um die Radikalfängereigenschaft von Caroverin mit ABTS zu vergleichen. In der Gegenwart von hochreaktiven Radikalen wird ABTS zu dem stabilen Thiazolin-Kationenradikal ABTS⁺ oxidiert. Die intensive grüne Farbe dieses Radikals wurde fotometrisch mit einer Hauptabsorptionsbande bei 420 nm vermessen. Caroverin war in der Lage, die Bildung des ABTS-Kationenradikals aufgrund der Hydroxyl- und Peroxylradikalbildung sowohl in Abwesenheit als auch in Anwesenheit von Gehirnhomogenat (Fig. 5) zu inhibieren. Caroverin reagierte hiebei schnell mit freien Hydroxyl-, Peroxyl- und ABTS-Kationenradikalen, wie dies bei Vergleichsstudien mit ABTS in Abwesenheit und in Anwesenheit von Hirnhomogenat gezeigt wurde. Die Geschwindigkeitskonstante für die Reaktion mit Hydroxylradikalen war 0,4 x 10¹⁰ M/l/s und jene für Peroxylradikale war 0,5 x 10¹⁰ M/l/s, wie dies durch vergleichskinetische Studien mit ABTS in der Abwesenheit oder in der Anwesenheit von Hirnhomogenat durchgeführt wurde (Fig. 6). Diese Ergebnisse zeigen, daß Caroverin die ABTS-Kationenradikalen-Bildung inhibiert und die ABTS-Kationenradikalen-Reduktion in der Gegenwart und in Abwesenheit von Hirnhomogenat katalysiert und somit als sehr wirksamer Elektronendonor bei niedrigen, mikromolaren Konzentrationen wirkt. Hiebei wurde, wie bereits erwähnt, die ABTS-Kationenradikalen-Bildung und -Reduktion verwendet, um die Einelektron-Transferreaktionen zu untersuchen, da diese Substanz aufgrund ihrer Farbe eine direkte Messung der Radikalbildung und -reduktion ermöglicht.

Bei Vergleichsstudien wurde festgestellt, daß Caroverin wenigstens dreimal so wirksam wie die bekannten Antioxidantien Ascorbat, Trolox und Glutathion ist. In bezug auf die Radikalfängereigenschaft von Caroverin wurde festgestellt, daß sie jene von Melatonin erreicht, welches der bis dato wirksamste Elektronendonor und Radikalfänger ist, der detektiert wurde. Aus diesem Grund wird angenommen, daß Caroverin die Reparatur von langlebigen, organischen Radikalen und beschädigten Biomolekülen erleichtern kann.

### Beispiel 9

5 Patienten, 2 weibliche und 3 männliche, welche an einer Typ I Allergie, nämlich Heuschnupfen, leiden, wurden bei Auftreten der allergischen Primärreaktionen, nämlich Niesen, Halskratzen, sowie Nasenausfluß mit Caroverin behandelt. Es wurde ihnen 3 mal täglich eine Tablette enthaltend je 150 mg Caroverin verabreicht.

Bei allen 5 Patienten konnte das Auftreten von Sekundärerscheinungen der allergischen Reaktion, nämlich Halsrötungen und dgl., vermieden werden. Auch die Primärreaktionen, wie Niesen, Nasenausfluß und dgl., waren nach 2-tägiger Verabreichung von Caroverin vollständig verschwunden, und es wurde nach Verschwinden der Primärreaktionen zur Aufrechterhaltung der Wirkung eine weitere Woche Caroverin 2 mal täglich in niedrigen Dosen verabreicht.

Zum Vergleich wurden 2 Patienten mit den üblichen Antihistaminen behandelt, wobei sowohl die Primärerscheinungen der Allergie, als auch insbesondere die Sekundärerscheinungen nicht sofort und nicht vollständig unterdrückt werden konnten, so daß die Patienten an ihrer Krankheit Heuschnupfen im wesentlichen so lange litten, als die Belastung mit den Verursachern, nämlich Gräserpollen, aufrechterhalten wurde. Auch konnte die Verabreichung des Medikamentes, solange Pollenflug beobachtet werden konnte, nicht abgesetzt werden.

Die Wirkung von Caroverin beruht im vorliegenden Fall auf seiner extrem hohen antioxidativen Wirkung und insbesondere auf seinen Radikalenfängereigenschaften, die durch die Histamine bzw. Cytokine erzeugten OH-Radikale bzw. ONOO-Radikale selektiv umsetzen und somit unschädlich machen. Aufgrund der Verabreichung von Caroverin wird der allergische Zyklus unterbrochen und es traten keine weiteren Reaktionen auf.

### Beispiel 10

Die antioxidative Wirkung und insbesondere die Wirkung in bezug auf die vorzeitige Alterung von Hautzellen wurde durch Herstellen einer Salbenformulierung, enthaltend 150 mg Caroverin pro 100 g Creme, untersucht. Als Cremenbasis wurde Glycerin verwendet; weitere pharmazeutisch wirksame Zusätze waren in der Cremenformulierung nicht enthalten.

Fünf weibliche Testpersonen im Alter zwischen 35 und 45 Jahren, die durchschnittlich eine trockene und teilweise bereits deutliche Alterungserscheinungen aufweisende Haut besaßen, verwendeten die auf Caroverin basierende Creme über einen Zeitraum von 4 Wochen, zweimal täglich, sowohl als Grundlage für ein Make-up als auch als alleinige Nachtcreme.

Bereits nach zwei Wochen waren insbesondere bei den jüngeren Testpersonen deutliche Verbesserungen des Hauterscheinungsbildes zu beobachten, wobei insbesondere Sonnenschäden der Haut sowie durch Oxidation hervorgerufene Hautrötungen deutlich gemildert wurden. Nach vier Wochen wurde darüberhinaus eine etwas straffere und glattere Haut beobachtet.

Auch die älteren Versuchspersonen zeigten nach vier Wochen ein klareres Hautbild, bei welchem insbesondere Rötungen und Sonnenschäden nahezu vollständig zum Verschwinden gebracht werden konnten.

### Beispiel 11

Verwendung von 1-(Aminoalkyl)-3-chinoxalin-2-on-Derivaten als Antagonist der metabotropen Gruppe-I-Glutamatrezeptoren im Ohr von Meerschweinchen.

Eine Gruppe von sechs Meerschweinchen wurde in das Ohr Hydroxyphenylglycerin als Rezeptoragonist injiziert, wobei der Rezeptor in der Weise reagiert, daß die Hörleistung der Meerschweinchen stark erhöht wird. Meerschweinchen, welchen Hydroxyphenylglycerin injiziert wurde, zeigen schon bei extrem leisen Geräuschen Angst, laufen nervös in den Käfigen herum und bei Geräuschen, welche den normalen Lärmpegel, der in einem pharmazeutisch-chemischen Labor herrscht, nicht überschreiten, wird bei einigen der Meerschweinchen bereits ein Verhalten beobachtet, welches auf Schmerzen des Tieres rückschließen läßt. Vier der sechs Meerschweinchen wurde in der Folge eine Dosis von 8 mg/kg Körpergewicht des Meerschweinchens in die Ohrvene injiziert und das Verhalten der Meerschweinchen beobachtet. Überraschenderweise hat sich gezeigt, daß sich die Meerschweinchen unmittelbar nach Verabreichung von Caroverin beruhigten und daß sie auch auf laute Geräusche, wie Klatschen, in ihrer unmittelbaren Nähe nicht mehr angstvoll reagierten. So liefen die Meerschweinchen, wenn direkt vor ihrem Käfig geklatscht wurde, nicht davon und versteckten sich nicht in ihrer Schlafhöhle. Demgegenüber blieben die zwei Meerschweinchen, welche kein Caroverin erhielten, extrem aufgeregt und beispielsweise Klatschen vor ihrem Käfig bewirkte bei ihnen, daß sie rasch in ihrem Käfig auf- und abliefen und deutliche Anzeichen von Schmerz und Nervosität zeigten. Diese Verhaltensauffälligkeiten der Meerschweinchen wurden auch durch Anbringen von Elektroden an den Ohren der Tiere beobachtet, aus deren Meßdaten problemlos abgelesen werden konnte, ob das Aussetzen der Tiere an unerwartete Geräusche eine Anregung bewirkte oder nicht. Auch die Meßdaten der Elektroden zeigten deutlich, daß nach Verabreichung von Caroverin an die Meerschweinchen eine Anregung nicht bzw. nur in extrem geringen Ausmaßen erfolgte.

Weiters wurde am narkotisierten Tier durch Anbringen von Mikroelektroden am Hörnerv der hemmende Einfluß von Caroverin auf die metabotropen Gruppe-I-Glutamatrezeptoren untersucht. Aus den Meßergebnissen an Versuchstieren nach Verabreichung von Caroverin und an Vergleichsversuchstieren, welchen kein Caroverin verabreicht wurde, konnte ersehen werden, daß die Erregbarkeit der metabotropen Gruppe-I-Glutamatrezeptoren durch die Verabreichung von Caroverin massiv unterdrückt wurde.

Aus diesen Versuchsergebnissen kann geschlossen werden, daß Caroverin und somit 1-(Aminoalkyl)-3-chinoxalin-2-on-Derivate metabotrope Glutamatrezeptoren im Ohr von Meerschweinchen antagonisieren können.

### Beispiel 12

Verwendung von 1-(Aminoalkyl)-3-chinoxalin-2-on-Derivaten zur Prävention von Hautkrebs bei Meerschweinchen.

Eine Gruppe von sechs Meerschweinchen wurde im Nackenbereich rasiert und einer intensiven UV-Bestrahlung über einen Zeitraum von wenigstens zwei Stunden täglich unterzogen. Die UV-Bestrahlung enthielt hiebei sowohl UV-A- als auch UV-B- und UV-C-Strahlenanteile. Vier der sechs Meerschweinchen wurde vor Beginn der ersten Bestrahlung auf den rasierten Nackenbereich eine hochkonzentrierte Lösung von Caroverin.Hydrochlorid gepinselt, wobei darauf geachtet wurde, daß die Verteilung möglichst gleichmäßig und über den gesamten bestrahlten Bereich erfolgte.

Nach zwei Wochen intensiver Bestrahlung zeigten die Meerschweinchen der Vergleichsgruppe deutliche Hautschäden, wie Verbrennungen, Rötungen und oberflächliche Hautläsionen, wobei bei Probennahmen bereits Krebszellen in den Gewebeproben gefunden werden konnten. Die vier mit der Caroverin-.Hydrochlorid-Lösung behandelten Meerschweinchen zeigten nach derselben Bestrahlungsdauer zwar eine gewisse Hautrötung, jedoch konnten in den entnommenen Gewebeproben keine Krebszellen nachgewiesen werden.

Ein analoger Versuch wurde mit 1-Diethylaminoethyl-3-(p-hydroxybenzyl)-1,2-dihydrochinoxalin-2-on durchgeführt, wobei das idente Ergebnis erzielt wurde.

Aus dem obigen ergibt sich, daß die erfindungsgemäß verwendeten 1-Diethylaminoethyl-3-chinoxalin-2-on-Derivate extrem wirksame Antioxidantien und Radikalfänger für Radiakle des Sauerstoff-Zellmetabolismus sind und daß sie aufgrund dieser Eigenschaften in der Lage sind, die durch freie Radikale hervorgerufenen Veränderungen der DNA, insbesondere von Krebs, hintanzuhalten, und somit zur Prävention insbesondere von Hautkrebs geeignet scheinen.

### Beispiel 13

Drei Patienten im Alter zwischen 19 und 75 Jahren, welche an Kolonkarzinom litten, wurde neben der üblichen Chemotherapie mit Irinothekam und Oxaliplatin für die vierwöchige Dauer der Chemotherapie zusätzlich Caroverin verabreicht,
wobei die Verabreichung am Tag 1 in erhöhter Dosis, nämlich intravenös in physiologischer Kochsalzlösung erfolgte, und an den weiteren Tagen durch Verabreichung von 3 x 3 Tabletten täglich unterstützt wurde.

Aufgrund der extrem hohen antioxidativen Wirkung von Caroverin konnte bei dieser Studie ein vollständiges Verschwinden des Kolonkarzinoms beobachtet werden. 2 Vergleichspatienten, welche nicht mit Caroverin behandelt wurden, zeigten bei der Chemotherapie die bekannte Verkleinerung des Karzinoms, jedoch konnte dieses nicht zum Verschwinden gebracht werden.

Weitere Versuche bei Patienten, welche an fortgeschritten Kopf- und Halstumoren litten, zeigten ähnliche Ergebnisse,
wobei bei diesen Patienten neben laufender Chemotherapie ebenfalls Caroverin verabreicht wurde. Es konnte neben einer erfolgreichen Palliation auch eine Reduktion der Tumorgröße erreicht werden. Ein vollständiges Verschwinden der Tumore konnte nicht erreicht werden.

Zusammenfassend ergibt sich, daß aufgrund der extrem hohen antioxidativen Wirkung von Caroverin freie Radikale des Sauerstoffzellmetabolismus aus dem System beseitigt werden und somit eine weitere ständige Veränderung der DNA, durch welche Krebs hervorgerufen wird, hintangehalten werden kann. Caroverin erscheint somit nicht nur zur Prävention von Krebs geeignet, sondern insbesondere zur Behandlung von einigen spezifischen Tumorenarten hoch wirksam.

## Patentansprüche

1. Verwendung von 1-(Aminoalkyl)-3-chinoxalin-2-on-Derivaten, insbesondere 1-Diethylaminoethyl-3-chinoxalin-2-on-Derivaten der Formel 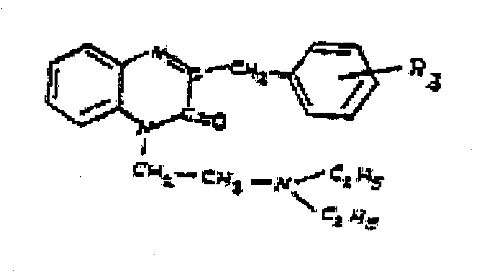 worin R₃ Hydroxy, Methoxy, Ethoxy oder Wasserstoff bedeutet, oder pharmazeutisch verträglichen Salzen davon, für die Herstellung von antioxidativen Zusammensetzungen zur Behandlung von durch freie Radikale des Sauerstoff-Zellmetabolismus hervorgerufenen Erkrankungen, gewählt aus der Gruppe von durch freie OH-Radikale hervorgerufenen Veränderungen der DNA, nämlich Krebs, von durch freie Radikale des Sauerstoff-Zellmetabolismus hervorgerufenen Zellalterungserscheinungen und/oder Zellzerstörungen, nämlich Hautalterungserscheinungen, von durch freie OH-Radikale und/oder freie ONOO-Radikale hervorgerufenen Typ 1-Allergien, und zur Stimulation des Wachstums von Nervenzellen, wie glutamatergen Nervenzellen, gewählt aus der Gruppe Wiederherstellung der Funktionsfähigkeit von Nerven nach einem Hirnschlag, Verbesserung von Schluckstörungen, Artikulationsstörungen, nämlich Sprachstörungen und Querschnittslähmungen, Behandlung von Schizophrenie oder der amyotrophen Lateralsklerose, Stimulation des glutamatergen Nervenwachstums bei einem Kochleaimplantat, Funktionsausfälle des Hörnervs, Gleichgewichtsnervs, Riechnervs oder Gesichtsnervs, wobei das 1-(Aminoalkyl)-3-chinoxalin-2-on-Derivat oder seine pharmazeutisch verträglichen Salze in einer täglichen Dosis von 5 bis 15 mg/kg Körpergewicht eingesetzt wird.

2. Verwendung nach Anspruch 1, worin als 1-(Aminoalkyl)-3-chinoxalin-2-on-Derivat das Methoxy-Derivat 1-Diethylaminoethyl-3-(p-methoxybenzyl)-1,2-dihydrochinoxylin-2-on oder seine pharmazeutisch verträglichen Salze eingesetzt werden.

3. Verwendung nach Anspruch 1, worin als 1-(Aminoalkyl)-3-chinoxalin-2-on-Derivat das Hydroxy-Derivat 1-Diethylaminoethyl-3-(p-hydroxybenzyl)-1,2-dihydrochinoxalin-2-on oder seine pharmazeutisch verträglichen Salze eingesetzt werden.

4. Verwendung nach der Anspruch 1, 2 oder 3, **dadurch gekennzeichnet, daß** das 1-(Aminoalkyl)-3-chinoxalin-2-on-Derivat oder seine pharmazeutisch verträglichen Salze in einer täglichen Dosis von 5 bis 10 mg/kg Körpergewicht eingesetzt werden.

5. Verwendung nach einem der Ansprüche 1 bis 4, **dadurch gekennzeichnet, daß** das 1-(Aminoalkyl)-3-chinoxalin-2-on-Derivat zur oralen, transdermalen, topischen, parenteralen oder intravenösen Verabreichung formuliert ist.

## Claims

1. The use of 1-(aminoalkyl)-3-quinoxaline-2-on derivatives and, in particular, 1-diethylaminoethyl-3-quinoxaline-2-on derivatives of the formula 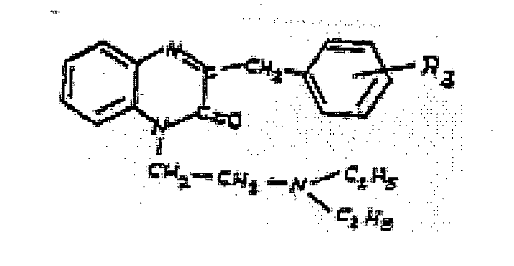 wherein R₃ is hydroxy, methoxy, ethoxy or hydrogen, or pharmaceutically acceptable salts thereof, for the preparation of antioxidative compositions for treating diseases caused by free radicals of the oxygen cell metabolism, selected from the group of DNA alterations produced by free OH radicals, namely cancer, of cell ageing manifestations and/or cell destructions, namely skin ageing manifestations, caused by free radicals of the oxygen cell metabolism, and/or of type I allergies produced by free OH radicals and/or free ONOO radicals, and for stimulating the growth of nerve cells, such as glutamatergic nerve cells, selected from the group for restoring the functionability of nerves after a cerebral stroke as well, for improving dysphagia, disturbed articulation, namely speech disorders and transverse lesions of the cord with paraplegia, for treating schizophrenia or amyotrophic lateral sclerosis, and for stimulating the glutamatergic nerve growth with a cochlear implant, functional deficits of the auditory nerve, balancing nerve, optic nerve, olfactory nerve or facial nerve, wherein the 1-(aminoalkyl)-3-quinoxaline-2-on derivative or its pharmaceutically acceptable salts are applied at a daily dose ranging from 5 to 15 mg/kg body weight.

2. The use according to claim 1, wherein the methoxy derivative 1-diethylaminoethyl-3-(p-methoxybenzyl)-1,2-dihydroquinoxaline-2-on or its pharmaceutically acceptable salts are used as said 1-(aminoalkyl)-3-quinoxaline-2-on derivative.

3. The use according to claim 1, wherein the hydroxy derivative 1-diethylaminoethyl-3-(p-hydroxybenzyl)-1,2-dihydroquinoxyline-2-on or its pharmaceutically acceptable salts are used as said 1-(aminoalkyl)-3-quinoxaline-2-on derivative.

4. The use according to claim 1, 2 or 3, **characterized in that** said 1-(aminoalkyl)-3-quinoxaline-2-on derivative or its pharmaceutically acceptable salts are applied at a daily dose ranging 5 to 10 mg/kg body weight.

5. The use according to any one of claims 1 to 4, **characterized in that** said 1-(aminoalkyl)-3-quinoxaline-2-on derivative is formulated for oral, transdermal, topical, parenteral or intravenous administration.

## Revendications

1. Utilisation de dérivés 1-(aminoalkyl)-3-quinoxalin-2-one, en particulier de dérivés 1-diéthylaminoéthyl-3-quinoxalin-2-one de la formule : 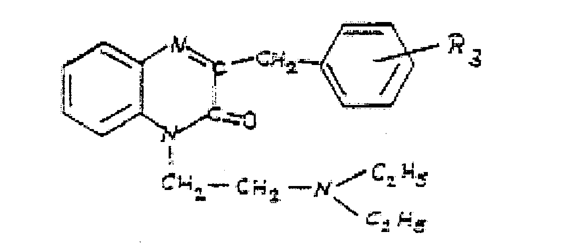 où R₃ représente hydroxy, méthoxy, éthoxy ou hydrogène, ou leurs sels pharmaceutiquement compatibles, pour la préparation de compositions antioxydantes pour le traitement de maladies engendrées par les radicaux libres du métabolisme cellulaire de l'oxygène, choisies parmi le groupe des modifications de l'ADN engendrées par des radicaux libres OH, à savoir le cancer, des phénomènes de vieillissement cellulaire et/ou de destruction cellulaire engendrés par les radicaux libres du métabolisme cellulaire de l'oxygène, à savoir les phénomènes de vieillissement de la peau, les allergies de type I engendrées par les radiaux libres OH et/ou les radicaux libres ONOO, et pour la stimulation de la croissance des cellules nerveuses, comme les cellules nerveuses glutaminergiques, choisies parmi le groupe du rétablissement de la capacité fonctionnelle des nerfs après une attaque cérébrale, l'amélioration du trouble de la déglutition, des troubles des articulations, à savoir troubles du langage et paraplégie, traitement de la schizophrénie ou de la sclérose latérale amyotrophique, stimulation de la croissance nerveuse glutaminergique lors d'un implant cochléaire, perte de fonction du nerf auditif, du nerf de l'équilibre, du nerf olfactif ou du nerf visuel, où le dérivé 1-(aminoalkyl)-3-quinoxalin-2-one ou son sel pharmaceutiquement compatible est mis en oeuvre selon une dose quotidienne de 5 à 15 mg/kg de poids corporel.

2. Utilisation selon la revendication 1, où l'on met en oeuvre comme dérivé 1-(aminoalkyl)-3-quinoxalin-2-one, la 1-diéthylaminoéthyl-3-(p-méthoxybenzyl)-1,2-dihydroquinoxalin-2-one ou son sel pharmaceutiquement compatible.

3. Utilisation selon la revendication 1, où l'on met en oeuvre comme dérivé 1-(aminoalkyl)-3-quinoxalin-2-one, le dérivé hydroxy 1-diéthylaminoéthyl-3-(p-hydroxybenzyl)-1,2-dihydroquinoxalin-2-one ou son sel pharmaceutiquement compatible.

4. Utilisation selon la revendication 1, 2 ou 3, **caractérisée en ce que** le dérivé 1-(aminoalkyl)-3-quinoxalin-2-one ou son sel pharmaceutiquement compatible est mis en oeuvre selon une dose quotidienne de 5 à 10 mg/kg de poids corporel.

5. Utilisation selon l'une des revendications 1 à 4, **caractérisée en ce que** le dérivé 1-(aminoalkyl)-3-quinoxalin-2-one est formulé pour une administration orale, transdermique, topique, parentérale ou intraveineuse.
